# EUROPEAN PATENT SPECIFICATION

(11) **EP 4 225 195 B1**
(45) Date of publication and mention of the grant of the patent: **15.10.2025**
(21) Application number: 21802406.5
(22) Date of filing: 22.09.2021
(51) Int. Cl.: A61B 34/20, A61B 17/24, G16H 15/00, G16H 30/20, G16H 40/67, A61B 90/00

(54) **PROCEDURE VISUALIZATION**
VISUALISIERUNG EINES VERFAHRENS
VISUALISATION DE PROCÉDURE

(30) Priority: 12.10.2020 US 202017068118
(43) Date of publication of application: 16.08.2023
(73) Proprietor: BIOSENSE WEBSTER (ISRAEL) LTD., 2066717 Yokneam (IL)
(72) Inventor: WOLFSON, Helen, 2066717 Yokneam (IL); SEGAL, Iris, 2066717 Yokneam (IL); PINSKY, Yoav, 2066717 Yokneam (IL); GUSEIN, George, 2066717 Yokneam (IL); HOD, Uriel, 2066717 Yokneam (IL)
(74) Representative: Carpmaels & Ransford LLP
(86) International application number: PCT/IB2021/058626
(87) International publication number: WO 2022/079524

(56) References cited:
- WO-A1-2018/195216
- US-A1- 2010 036 676
- US-A1- 2020 268 472
- ANONYMOUS: "Instructions for Use Fiagon Navigation Software Version 3.7 Model ENT", 1 January 2018 (2018-01-01), pages 1 - 38, XP055877298, Retrieved from the Internet <URL:https://www.intersectent.com/ifu/Fiagon-Navigation-Software-ENT-IFU-Instructions-for-Use.pdf> [retrieved on 20220110]
- BRAINLAB: "Digital O.R. Customer Stories - Grosshadern Clinic at Ludwig-Maximilians-University Hospital, Munich", 21 December 2017 (2017-12-21), XP055877318, Retrieved from the Internet <URL:https://www.youtube.com/watch?v=9bHT9J6x8Kc&list=PLa8CkGDnfTl5PlMYSxYBRwg5KWRqBh3pC&index=5> [retrieved on 20220110]

## Description

### FIELD OF INVENTION

The present application provides systems, apparatuses, and methods for improving medical procedures.

### BACKGROUND

Visualization of internal body structures and tracking of probes can be performed by mapping propagation of activation waves, tracking electromagnetic components, and the like. Fluoroscopies, computerized tomography (CT), ultrasound and magnetic resonance imaging (MRI), as well as other techniques may be utilized to provide the visualization and graphical renderings of intra-body structures.

Ear, nose, and throat (ENT) physicians are commonly provided with attributes, visualizations, and details regarding a given procedure as the procedure is being performed. However, such attributes, visualizations, and details are currently limited in scope (e.g., limited to information available only at a given point in time). Currently, ENT physicians may only analyze a procedure in real time such that no log or record of the procedure is available for subsequent analysis. Additionally, no historical input is available to a physician while performing a procedure.

Document XP055877298 "Instructions for Use Fiagon Navigation Software Version 3.7 Model ENT" discloses taking screenshots and record video sequences for a documentation of the operation using a probe. Document WO 2018/195216 A1 discloses snapshots saved during a medical procedure.

### SUMMARY

Apparatus and methods are provided for receiving an image of a portion of a body anatomy of a patient, registering the image to patient coordinates, inserting a probe into the portion of the body anatomy, the probe comprising a tool tip configured to identify the position of the probe relative to the registered image, performing the procedure, the procedure comprising navigating the probe within the portion of the body anatomy, generating a procedure report comprising one or more components related to the procedure, the one or more components comprising a visual indication of areas of the portion of the body anatomy navigated to by the probe, and providing the procedure report in a selected format upon termination of the procedure.

The present invention is defined by appended claims 1 and 14. Specific embodiments are set forth in the dependent claims.

### BRIEF DESCRIPTION OF THE DRAWINGS

A more detailed understanding can be had from the following description, given by way of example in conjunction with the accompanying drawings.
FIG. 1 is a diagram of an exemplary system in which one or more features of the disclosed subject matter can be implemented;
FIG. 2 is a graphic representation of a probe;
FIG. 3 is a graphic representation of a probe's path during a procedure;
FIG. 4 is a process for providing a procedure report in a selected format;
FIG. 5 is an example simple procedure report according to a selected format;
FIG. 6A is an example detailed procedure report according to a selected format;
FIG. 6B is another portion of the example detailed procedure report according to the selected format of FIG. 6A;
FIG. 6C is another portion of the example detailed procedure report according to the selected format of FIG. 6A;
FIG. 7 is a visual snapshot of a patient's anatomy as provided via a procedure report;
FIG. 8 is a block diagram of an example system for remotely monitoring and communicating patient biometrics; and
FIG. 9 is a system diagram of an example of a computing environment, which includes the computing system shown in FIG. 8.

### DETAILED DESCRIPTION

According to implementations of the disclosed subject matter, intra body medical procedures (e.g., Ear, Nose, and Throat (ENT) procedures) may be performed by a medical professional and one or more procedure reports may be generated based attributes of the performed procedure. A procedure report may be generated by transforming data generated based on physical movement, body function, and/or actions taken or their responses into a user understandable format. The procedure report may be based on historical data, one or more recognized standards, and may further be based on an artificial intelligence (e.g., machine learning) output, as further disclosed herein. Aspects of the generated procedure report may provide confirmation or insight regarding one or more given outcomes of a respective cases. In general, the proposed procedure reports may be utilized to provide detailed information to patients and physicians regarding the outcomes of medical procedures, such as ENT procedures, provide feedback to physicians in relation to their level of proficiency with the a given medical system. The procedure reports, as disclosed herein, may include visual representation of a patient's anatomy as medical devices such as medical probes interact with the anatomy.

Notably, the procedure reports, as disclosed herein, may provide detailed procedure specific information that is otherwise traditionally only available to a physician during a given procedure. Such detailed procedure specific information may help identify next steps, confirm portions of a procedure, and enable a medical professional to evaluate the procedure in a manner that is not otherwise possible during the procedure itself.

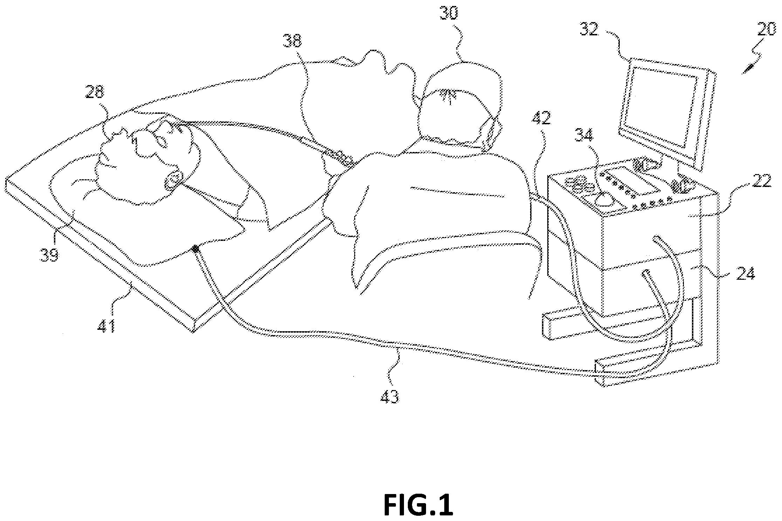

FIG. 1 is a diagram of an exemplary medical system 20 in which one or more features of the disclosure subject matter can be implemented. The medical system 20 includes a data processing component 22 and a data storage component 24 configured to process and store 3D scan images. For example, a 3D scan image derived from imaging data of a scan of a head 26 of a patient 28 for whom an ENT (ear/nose/throat) physician 30 is to perform a non-invasive surgical operation at a selected site within the patient's head 26.

The medical system 20 includes a monitor or other display device 32 for selectively displaying, for example, selected cross-section or perspective views from the 3D scan image of the patient's head 26. The data processing component 22 can include one or more CPUs, GPUs and/or other processors that are coupled to the data storage component 24 and display device 32 to produce desired cross-section or perspective views on the display device 32 from a 3D scan image, such as derived from scan data of the patient's head 26.

The medical system 20 includes one or more peripheral devices, such as a track ball and/or touch pad 34, to permit a user to select a particular view of, for example, the 3D imaging data of the scan of head 26 to display on the display device 32. On or more of the peripheral devices, such as device 34, is also configured to permit a user to select specific voxels which serve as end points of a pathway therebetween. Such peripheral devices can include, but are not limited to, computer mouse devices, video gaming controller devices, joystick devices, laser pointing devices, voice command devices and touch screen display controls. Preferably, one or more peripheral devices are employed to permit the user, such as the ENT physician 30 or a surgical assistant, to pan through consecutive views of the 3D scan image as may be desired an to select a target voxel at which to deploy a surgical tool to perform a surgical procedure.

The medical system 20 may also include devices, such as a catheter 38 which includes a surgical tool or through which a surgical tool can be inserted for operation thereof at a distal end of the catheter 38. The catheter 38 can include ultrasound transducer configured to obtain biometric data or ultrasound slices or the like. The distal end of the catheter 38 can include a probe that may operate in connection with a location pad 39 the disposed on a gurney 41 which the patient 28 is placed for the surgical procedure.

In the illustrated example of FIG. 1, the distal end probe of the catheter 38 and the location pad 39 comprise location sensing equipment and are coupled to the processing component 22 by respective cables 42 and 43. In this example, the processing component 22 is configured to use signals from the catheter probe and location pad 29 to track the location of the distal end of the catheter 38 as it is inserted by the ENT physician 30 into the subject's cranium and to display a visualization of the catheter travel on the display device 32 in connection with the view of the 3D scan image being displayed.

Although an ENT physician 30 is shown in the example provided in FIG. 1, it will be understood that any medical professional may replace ENT physician 30 in the example of FIG. 1.

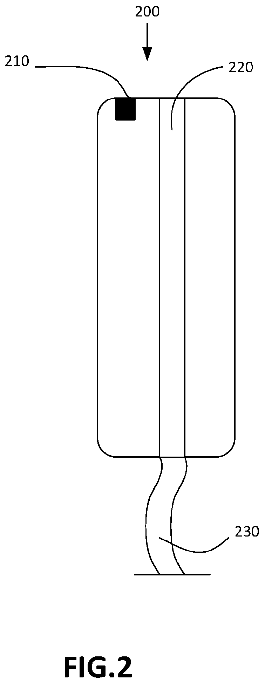

As noted herein, the distal end of the catheter 38 can include a probe. An example probe 200 is shown in FIG. 2. The probe 200 comprises a tool tip 210 which may be configured to provide location information. For example, tool tip 210 may transmit electromagnetic signals that are then received by external receivers (e.g., the location pad 39 of FIG. 1) such that the location of the probe 200 can be determined based on the electromagnetic signals. Alternatively, tool tip 210 may receive electromagnetic signals from one or more external components and may generate the location of the probe 200 based on the electromagnetic signals.

The location of the probe 200 may be used to track the areas of a portion of a body anatomy navigated to by the probe. Such tracked areas may be provided, via a procedure report, as further disclosed herein. For example, the areas of a portion of a body anatomy navigated to by the probe may be provided using a visual indication overlaid on a representation of the portion of the body anatomy.

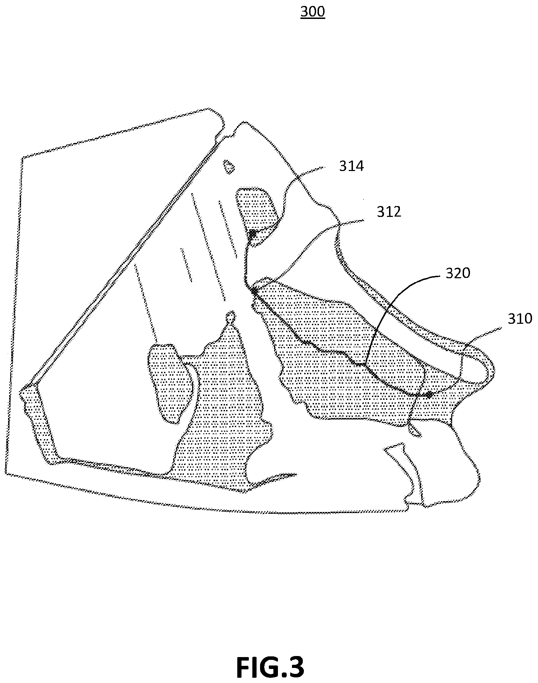

FIG. 3 shows an example representation of a portion of a body anatomy 300. A probe such as probe 200 of FIG. 2 may be inserted into the portion of the body anatomy 300 of FIG 3, and may be navigated across points 310, 312, and 314 within the body anatomy 300. A procedure report may be generated and may include a visual representation 320 overlaid on a rendering of the portion of the body anatomy 300. The visual representation 320 may be an indication of the areas and/or route that the probe 200 navigated through the body anatomy 300. The visual representation 320 may be provided based on a tool tip (e.g., tool tip 210 of FIG. 2) used to determine location information of the probe 200 as it navigates through the body anatomy 300.

Additionally, the probe 200 of FIG. 2 may be configured to implement one or more other tasks. For example, a lumen 220 may be provided within the probe 200 such that an item (e.g., a needle, coils, etc.) can pass through the lumen 220. For example, coils (e.g., platinum coils) may be inserted via a catheter (e.g., catheter 38 of FIG. 1) through shaft 230 of FIG. 2 and into the probe 200. The coils may traverse through the probe 200 via lumen 220 and may exit from the distal end of the probe 200 away from shaft 230 and inserted into a patient's body.

According to implementations of the disclosed subject matter, an endoscopic medical procedure such as an ENT endoscopic sinus surgery procedure may be performed by a medical professional (e.g., ENT physician 30 of FIG. 1). The medical procedure may performed by using an electromagnetic image-guided navigation system designed for intranasal and/or paranasal image-guided navigation. For example, the electromagnetic image-guided navigation system may be used to treat chronic sinusitis.

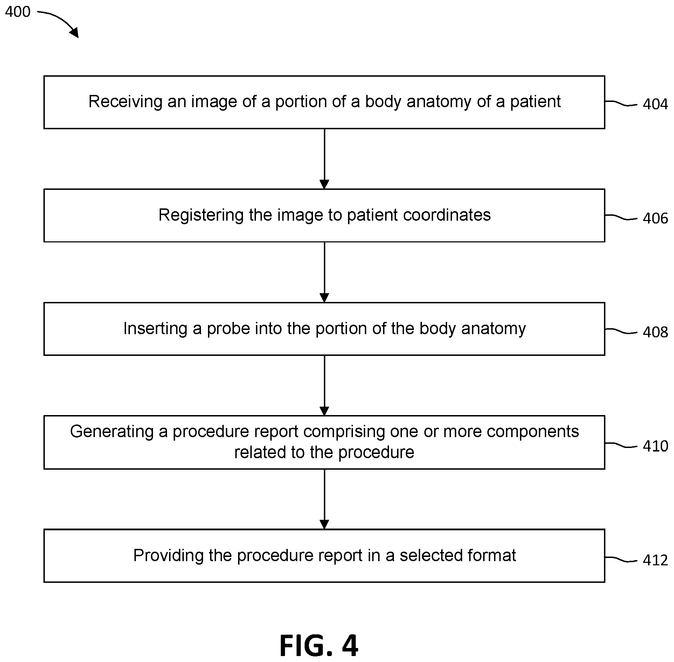

FIG. 4 shows a process 400 for generating a procedure report in accordance with implementations of the disclosed subject matter. At 404 of the process 400, an image of a portion of the body anatomy of a patient may be received. For example, the image may be a computed tomography (CT) image or a magnetic resonance (MR) image. The image of the portion of the patient's anatomy may be captured at a first time such as prior to a medical procedure.

According to an implementation, the image of the portion of the patient's anatomy may be qualified for use based on automatic identification of features. For example, a scan of the image may be conducted to determine if known anatomical features that can function as one or more fiducials are recognizable in the image. An image that is distorted or has low resolution may not be qualified for use in the medical procedure as the known anatomical features may not be easily visible in the application. An image score may be determined for the image such that an image with an image score below a threshold may not qualify for use in the medical procedure.

At step 406 of the process, the image of the portion of the patient's anatomy may be registered to patient coordinates prior to or while the medical procedure is initiated. Corresponding points in the portion of the patient's anatomy and the image of the portion of the patient's anatomy are aligned during the registration process. The registration process may be implemented by a computing component, such as data processing component 22, of FIG. 1 which may be internal or external to a medical system such as medical system 20. During the registration process, one or more aspects of the image of the portion of the patient's anatomy such as a body part, a curve, a point, or the like may be matched with one or more patient coordinates as the patient is positioned for the procedure. As an example, a patient's eye sockets may be matched with the eye socket bone structure provided in the image (e.g., a CT image) and such that the coordinates represented by the patient's eye sockets are matched to the corresponding bone structure in the image and the image is registered to the patient's coordinates.

According to an implementation, the image (e.g., a CT image) of the portion of the patient's anatomy may be registered to the patient coordinates using a scanning device that may scan a patient's body externally. One or more features of the patient's anatomy may be detected using the external scan of the patient's body. The one or more features may be aligned with the image of the portion of the body anatomy of the patient, as received at step 404. For example, a patient's nose may be scanned by an external camera and the scan may be registered with the image of patient.

At step 408 of the process 400, a probe, such as probe 200 of FIG. 2, may be inserted into the patient. Notably, the probe may be inserted into the portion of the body anatomy that corresponds to the image received at step 404. The probe may be inserted via a laparoscopic technique, an endoscopic technique, or via any applicable technique that enables the probe to reach the inside of the patient's body.

The probe may be inserted at step 408 and may be tracked such that the location of the probe as it traverses the inside of the patient's body is tracked via any applicable means. For example, one or more magnetic and/or electrical sensors on the probe may transmit a signal that is received by a tracking system (e.g., a location pad) and the tracking system may determine the coordinates of the probe based on the magnetic and/or electrical signals transmitted by the sensors. The coordinates may be mapped to the image of the portion of the body anatomy received at step 404 based on the registration of the image to the patient coordinates at step 406.

The probe inserted into the patient's body may be used to perform a procedure from any number of procedures or aspects of procedures. For example, the probe may be used to map a portion of the patient's anatomy, to perform ablation, to biopsy a portion of the patient's body or organ, to clear fluid, to insert a stent, or any other applicable use of the probe. The procedure may be operator (e.g., doctor) guided or may be a fully or partially automated procedure. According to an implementation, the procedure may be conducted by a remote operator such that the remote operator may provide guidance via an input device from a first location and the guidance may be electronically translated to control the operation of the probe at a second location. Notably, the probe may traverse numerous points within the patient's anatomy such that multiple points of interest are traversed during operation of the probe.

At step 410 of process 400, a procedure report including one or more components related to the procedure may be generated. The procedure report may have a type such that multiple types of procedure reports (e.g., simple procedure report, detailed procedure report, etc.) may be available based on a given procedure. A type of procedure report from a plurality of available types of procedure reports may be selected by an operator, a predetermined setting, a setting determined based on historical operator preference, or the like.

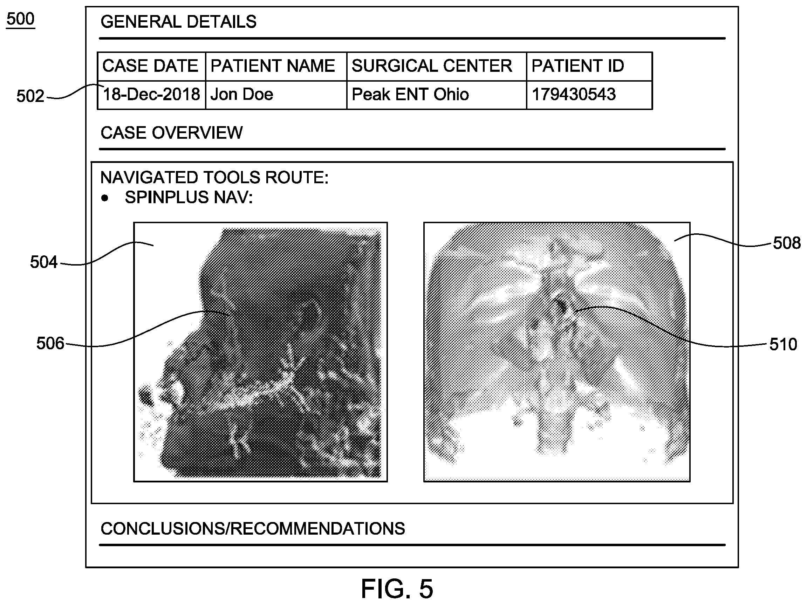

Implementations of the disclosed subject matter provide procedure reports based on a given medical procedure. A procedure report may include a plurality of components within the report. FIG. 5 shows a simple procedure report 500, according to an implementation of the disclosed subject matter. As shown in FIG. 5, the simple procedure report 500 includes patient information 502 such as a case date, patient name, surgical center, patient ID, and the like. Additionally, the simple procedure report 500 may include a high level case overview that includes a side view 504 of a patient's anatomy along with areas 506 traversed by a probe. Similarly, the simple procedure report 500 may include a front view 507 that shows areas 510 visited by the probe. According to an implementation, the areas 506 and areas 510 may be the same areas observed form a different perspective.

According to an implementation, the simple procedure report 500 may be may be provided to a patient. Alternatively, a detailed procedure report may also be generated based on a given procedure. The detailed procedure report may be a full procedure report, for use by a medical professional (e.g., for procedure evaluation, for training, etc.). The medical professional or other entity may also choose which details to include in a given reports (e.g., a simple report, a detailed report, etc.). The medical professional or other entity may select the details for a given report either prior to, during, or after a given procedure. According to an implementation, the details to include in a given report may be determined dynamically (e.g., based on the procedure itself, attributes of the patient, or attributes of the medical professional) and/or based on stored preferences.

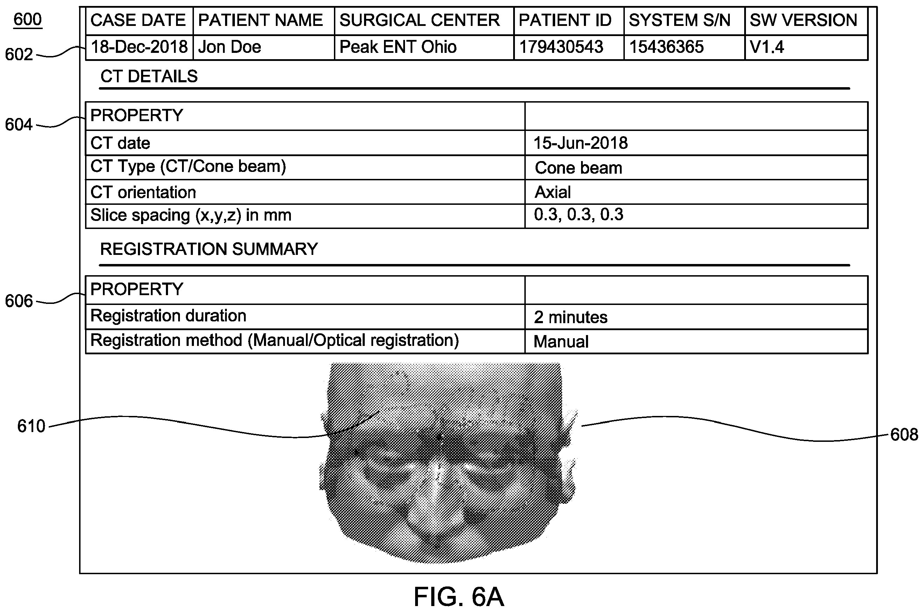

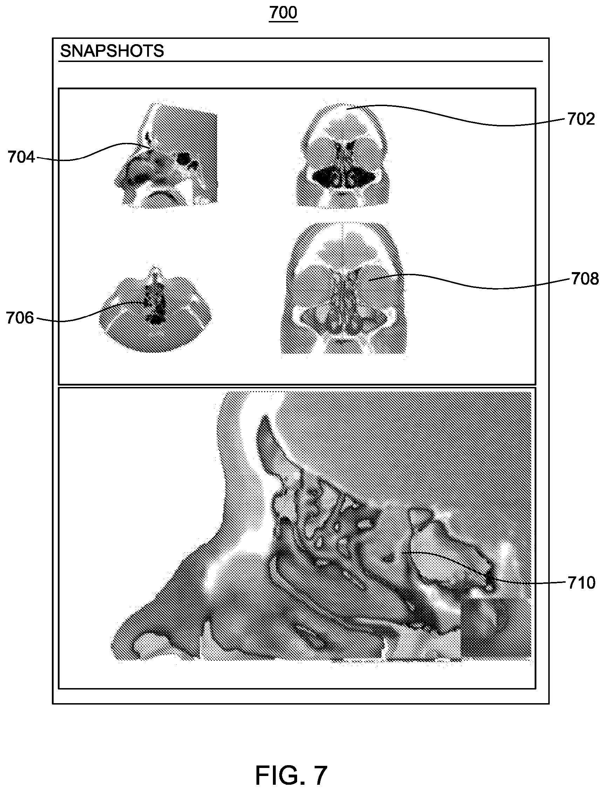

FIGs. 6A-6C show a detailed procedure report, according to an implementation of the disclosed subject matter. In particular, as shown in FIGS. 6A-6C, a detailed report may include patient information 602 such as a case date, patient name, surgical center, patient ID, and the like as well as system information and software version (i.e., information in addition to the simple procedure report 500). Additionally, the detailed report may include data regarding the preoperative CT scan at 604, registration procedure information at 606, navigation procedure information 610 in view 608, the type of ENT tools used at 620, a timeline 622, a 3D visual case overview 624 with time markers at 626, visual snapshots 700 of FIG. 7, and conclusions/recommendations. Notably, the detailed procedure report includes information above and beyond what is included in the simple procedure report.

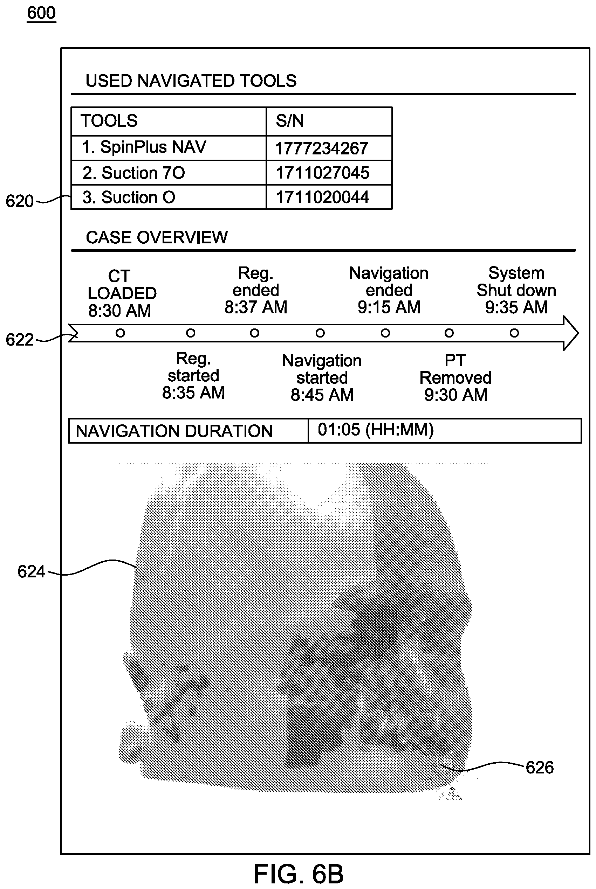

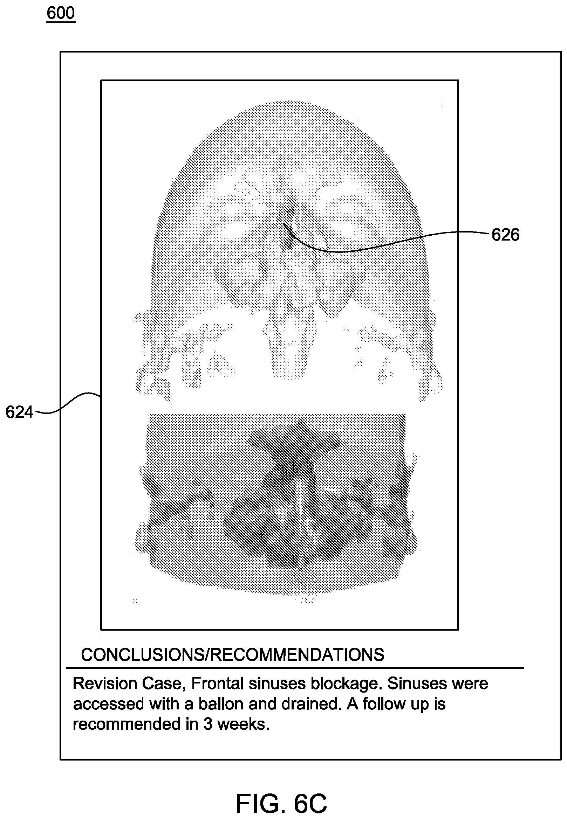

According to an implementation of the disclosed subject matter, a detailed procedure report may enable a medical professional to post operatively observe aspects of a given procedure that may not be available to the physician during the procedure or may otherwise be inefficient to access during a given procedure. According to an embodiment of the invention, as shown in FIG. 6B and FIG. 6C, a 3D visual case overview 624 is provided via a detailed procedure report. The 3D visual case overview 624 enables a medical professional to manipulate the procedure report to view and/or evaluate aspects of the given procedure after the procedure has been completed. A medical professional is enabled to rotate through the different views of the patient anatomy via the 3D visual case overview 324 such that the probe and/or the patient anatomy is visible to the medical professional from different viewpoints.

According to an implementation of the disclosed subject matter, as shown in FIG. 6B, a procedure report may be provided using a timeline format 622 that is segmented based a user action or a system status. The timeline format may be interactive such that a user may be able to interact with the timeline to review different aspects of the procedure via the procedure report. Each event in the timeline may correspond to a user action (e.g., in the user action format) or a system status (e.g., in the system status format).

As shown in FIG. 7, the procedure report may include snapshots of the patient's anatomy captured during the procedure. Alternatively, the procedure report may include snapshots of the surgical device's monitor, which can be taken at any point in time during the procedure. The monitor may include additional features additional to the anatomy; such as: VR, planning points, virtual cameras, segmentation, FAM, etc. These are all superimposed on the anatomy. The snapshots may include various views such as the views 702, 704, 706, and 708, as shown in FIG. 7. The various views may be available at various points of the procedure such as at different timeline segments, as described herein. As also shown in FIG. 7, at 710, a user (e.g., medical professional) may be able to interact with (e.g., zoom in and out) the procedure report to via snapshots in more or less detail.

According to an implementation, a user (e.g., medical professional) may select the type (e.g., user action or system status) of timeline format 622 to use when viewing a procedure report. The user may be able to switch between different types of timeline formats at any time by making an interactive selection via the procedure report.

A timeline format segmented based on user action may include providing a set of data corresponding to a point in time during the procedure such that the point in time is determined by a selected user action from a plurality of user actions during the procedure. The user action may be anything that is done by a medical professional or other operator. As an example, the user action may be a button press, a navigational adjustment made, a force applied, a marker placed (e.g., such that the given time can be recorded in the procedure report), an incision or cut made, or the like.

Accordingly, a timeline format segmented based on user action may enable a user (e.g., medical professional) to observe procedure characteristics based on changes in user action. For example, a physician may want to observe the quality of a contact between a probe and the patient's tissue. Accordingly, the physician may select a change in direction of the probe as a user action to observe the contact.

A timeline format segmented based on system status may include providing a set of data corresponding to a point in time during the procedure such that the point in time is determined by a selected system status from a plurality of system statuses during the procedure. The system status may be any status update, change, or lack of change as recorded during the procedure. FIG. 6B's timeline format 622 shows an example timeline format segmented based on system statuses. As shown, the timeline format may be segmented based on one or more of a CT being loaded, a registration starting and ending, a navigation starting or ending, a probe removal, a system shut down, or the like.

According to an implementation, a medical professional may review the effect of a given action (e.g., applying force via a probe) at a given location during a first portion of the procedure, as reviewed via a procedure report at the end of the procedure. To clarify, a given action (e.g., applying force via a probe at a given location) may be taken at a first time during a procedure. A procedure report may enable a medical professional to observe the effect of the given action at a second subsequent time, prior to the completion of the procedure. This implementation may enable a physician to receive fast feedback regarding actions taken during the procedure without extending the duration of the procedure to re-evaluate the result of the given action during the procedure itself.

The procedure reporting system described herein enables visualization of all the locations traversed during a given procedure (e.g., the frontal sinus). Such a visualization may enable a medical professional, who is otherwise unsure whether a particular point was traversed during the navigation procedure, to confirm that the particular point was covered during the procedure. Such a confirmation may also provide confirmation for a third party such as a patient, a different medical professional, an insurance company, or the like.

According to an implementation, a procedure report may also provide detailed information about the locations traversed during a procedure. For example, as shown at 610 in FIG. 6A, the locations traversed by a probe during a procedure are provided as indicators (e.g., dots as shown in FIG. 6A) . According to an implementation, the indicators may be colored or use another attribute (e.g., shape, size, pattern, etc.) to indicate a given property of the probe or the patient while the probe was at the given location.

As an example, the indicators may have attributes that indicate the length of time that the probe spent at the given location. As another example the indicators may have attributes that indicate the contact force with the tissue at the given location. As another example the indicators may have attributes that indicate an action performed by the probe at the given location. As another example the indicators may have attributes that indicate the response of tissue based on the probe being located at the given location.

According to an implementation, the detailed procedure report may include the visual indicators corresponding to the areas of the patient's anatomy that the probe traversed, as disclosed herein. Additionally, the procedure report may compare the visual indicators to a previously captured visualization of the patient anatomy (e.g., including a problematic area of the anatomy). The procedure report's visual indicators may be overlaid on the previously captured visualizations such that the procedure report may confirm whether or not the problematic area of the anatomy was traversed with the probe during a given procedure.

According to an implementation, the procedure reporting system disclosed herein may provide feedback based on the given procedure that a procedure report is generated based on. For example, to evaluate the accuracy of a particular ENT registration procedure, the system may also include the registration data compared to a physician's prior procedures, to the registration procedures performed by other physicians, and/or to recognized standards. Notably, a procedure report in accordance with the disclosed subject matter may be generated to include a visual or other applicable comparison to a physician's prior procedures, to the registration procedures performed by other physicians, and/or to recognized standards. Such a comparison may be used by the physician or by the patient to evaluate the quality of a given procedure.

Alternatively, or in addition, the comparison may be used to determine next steps. For example, a comparison that indicates a deviation from a physician's prior procedures, from the registration procedures performed by other physicians, and/or from recognized standards may enable a physician to determine whether a subsequent procedure is required. Alternatively, a successful procedure that deviates from a physician's prior procedures, to the registration procedures performed by other physicians, and/or to recognized standards may enable a physician to document an improved technique for conducting a procedure.

According to an implementation, the procedure report may be used to provide guidance to training professionals to learn the workflows for particular procedures (e.g., ENT procedures). According to this implementation, a training physician may perform a given procedure on a patient, a cadaver, or a simulated body (e.g., either a physical replica of a body or an electronically generated body). The performed procedure may then be compared, via a procedure report, to an experienced physician's prior procedures, to the registration procedures performed by other physicians in the industry, and/or to recognized standards. Based on the comparison, a training physician may be able to evaluate his or her success proficiency with a given technique, with equipment, with a workflow, or the like.

As disclosed herein, the procedure reporting system disclosed may include detailed information of similar procedures as conducted by other medical professionals. A procedure report may include a comparison of a given procedure to an average of all similar procedures conducted by other medical professionals. For example, the procedure reporting system may obtain procedure data for a plurality of procedures tagged as the same procedure as a given procedure for which a procedure report is being generated. The system may review an aspect of all the procedures (e.g., locations traversed by a probe for each procedure) and generate an average or otherwise representative version of the aspect. A procedure report generated for the given procedure may include a comparison of the same aspect for the given procedure to the average or representative version of the aspect as generated based on other procedures.

According to an implementation, a procedure report may be generated to include a comparison to other procedures, as disclosed, such that the other procedures are narrowed based on one or more factors. For example, a procedure report may be generated based on all the geographic locations where the same procedure as a given procedure is being performed. Alternatively, a user (e.g., medical professional, administrator, etc.) may narrow the comparison such that a subset of geographic locations are selected (e.g., based on the geographic background of the patient) and compared to the given procedure. It will be understood that although geographic locations are provided as an example, the number of procedures that are used for comparison may be narrowed based on any applicable factor such as patient demographics, equipment used, time period, medical history, anatomy features, the type of medical condition, or the like. Such comparisons may be used to improve patient outcomes by providing real-time feedback and guidance to a physician in relation to best practices for specific parts of a procedure, via a post procedure report.

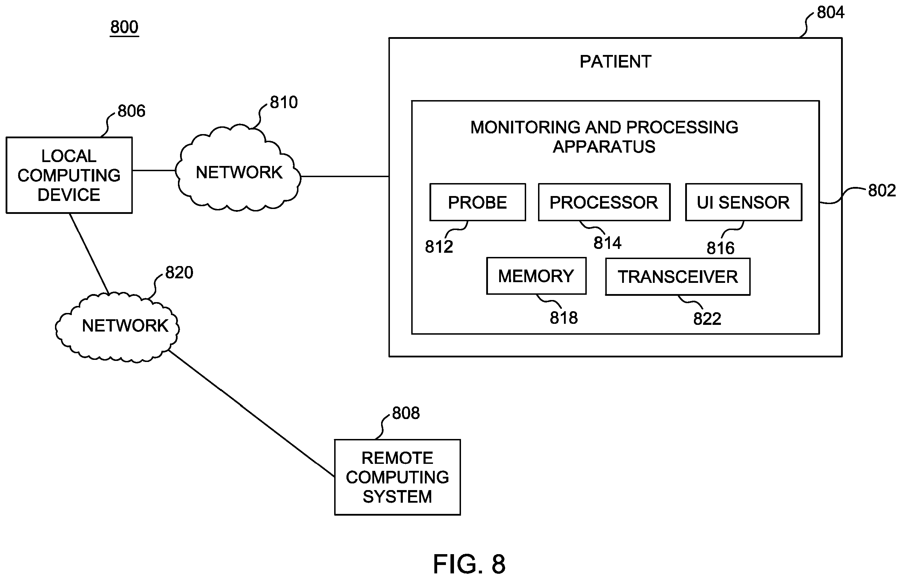

According to an implementation of the disclosed subject matter, a procedure report (e.g., in the selected format) may be provided via remote monitoring such that procedure data to generate the procedure report is transmitted to a location remote from the actual procedure. FIG. 8 is a block diagram of an example system 800 for remotely monitoring and communicating procedure reports. In the example illustrated in FIG. 8, the system 800 includes a patient monitoring and procedure apparatus 802 associated with a patient 804, a local computing device 806, a remote computing system 808, a first network 810 and a second network 820.

According to an embodiment, a monitoring and procedure apparatus 802 may be an apparatus that is internal to the patient's body (e.g., subcutaneously implantable). The monitoring and procedure apparatus 802 may be inserted into a patient via any applicable manner including orally injecting, surgical insertion via a vein or artery, an endoscopic procedure, or a laparoscopic procedure.

According to an embodiment, a monitoring and procedure apparatus 802 may be the probe 38 of FIG. 1 and/or any associated devices such as catheters, electrodes, needles, suction devices, or the like. Additionally, a monitoring and procedure apparatus may include additional devices such as the data processing component 22 and a data storage component 24 of FIG. 1. According to an embodiment, a monitoring and procedure apparatus 802 may include both components that are internal to the patient and components that are external to the patient.

A single monitoring and procedure apparatus 802 is shown in FIG. 8. Example systems may, however, may include a plurality of patient monitoring and procedure apparatuses. A patient monitoring and procedure apparatus may be in communication with one or more other patient monitoring and procedure apparatuses. Additionally, or alternatively, a patient monitoring and procedure apparatus may be in communication with the network 810.

One or more monitoring and procedure apparatuses 802 may acquire patient data during a procedure, as disclosed herein, and receive at least a portion of the patient data and additional information from one or more other monitoring and procedure apparatuses 802. The additional information may be, for example, diagnosis information and/or additional information obtained from an additional device such as a wearable device. Each monitoring and procedure apparatus 802 may process data as well as data received from one or more other monitoring and procedure apparatuses 802.

In FIG. 8, network 810 is an example of a short-range network (e.g., local area network (LAN), or personal area network (PAN)). Information may be sent, via short-range network 810, between monitoring an processing apparatus 802 and local computing device 806 using any one of various short-range wireless communication protocols, such as Bluetooth, Wi-Fi, Zigbee, Z-Wave, near field communications (NFC), ultraband, Zigbee, or infrared (IR).

Network 820 may be a wired network, a wireless network or include one or more wired and wireless networks. For example, a network 820 may be a long-range network (e.g., wide area network (WAN), the internet, or a cellular network,). Information may be sent, via network 820 using any one of various long-range wireless communication protocols (e.g., TCP/IP, HTTP, 3G, 4G/LTE, or 5G/New Radio).

The patient monitoring and procedure apparatus 802 and/or associated or connected devices may include a patient biometric sensor 812 (e.g., an intrabody probe), a processor 814, a user input (UI) sensor 816, a memory 818, and a transmitter-receiver (i.e., transceiver) 822. The patient monitoring and procedure apparatus 802 may continually or periodically monitor, store, process and communicate, via network 810, any number of various patient procedure details. Examples of patient procedure details include electrical signals (e.g., brain biometrics, voltages), location tracking signals, impedance measurements, temperature data, etc.

As stated, the patient monitoring and procedure apparatus 802 may be an ENT probe. The patient biometric sensor 812 of the ENT probe may include one or more electrodes for acquiring electrical signals. In other types of procedures, such as a cardiac procedure, EGC signals may be used for analyzing and/or treating a condition during a given procedure and/or during a follow-up procedure.

Transceiver 822 may include a separate transmitter and receiver. Alternatively, transceiver 822 may include a transmitter and receiver integrated into a single device.

Processor 814 may be configured to store patient data, such as patient biometric data in memory 818 acquired by patient biometric sensor 812, and communicate the patient data, across network 810, via a transmitter of transceiver 822. Data from one or more other monitoring and procedure apparatus 802 may also be received by a receiver of transceiver 822, as described in more detail below.

According to an embodiment, the monitoring and procedure apparatus 802 includes UI sensor 816 which may be, for example, a piezoelectric sensor or a capacitive sensor configured to receive a user input, such as a tapping or manipulation of a touch pad. For example UI sensor 816 may be controlled to implement a capacitive coupling, in response to tapping or touching a surface of the monitoring and procedure apparatus 802 by the patient 804. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface such that the tapping or touching of the surface activates the monitoring device.

The processor 814 may be configured to respond selectively to different tapping patterns of the capacitive sensor (e.g., a single tap or a double tap), which may be the UI sensor 816, such that different tasks of the patch (e.g., acquisition, storing, or transmission of data) may be activated based on the detected pattern. In some embodiments, audible feedback may be given to the user from processing apparatus 802 when a gesture is detected.

The local computing device 806 of system 800 is in communication with the patient biometric monitoring and procedure apparatus 802 and may be configured to act as a gateway to the remote computing system 808 through the second network 820. The local computing device 806 may be, for example, a, smart phone, smartwatch, tablet or other portable smart device configured to communicate with other devices via network. Alternatively, the local computing device 806 may be a stationary or standalone device, such as a stationary base station including, for example, modem and/or router capability, a desktop or laptop computer using an executable program to communicate information between the processing apparatus 802 and the remote computing system 808 via the PC's radio module, or a USB dongle. Patient biometrics may be communicated between the local computing device 806 and the patient biometric monitoring and procedure apparatus 802 using a short-range wireless technology standard (e.g., Bluetooth, Wi-Fi, ZigBee, Z-wave and other short-range wireless standards) via the short-range wireless network 810, such as a local area network (LAN) (e.g., a personal area network (PAN)). In some embodiments, the local computing device 806 may also be configured to display the acquired patient electrical signals and information associated with the acquired patient electrical signals, as described in more detail below.

In some embodiments, remote computing system 808 may be configured to receive at least one of the monitored patient procedure data and information associated with the monitored patient via network 820, which is a long-range network. For example, if the local computing device 806 is a mobile phone, network 820 may be a wireless cellular network, and information may be communicated between the local computing device 806 and the remote computing system 808 via a wireless technology standard, such as any of the wireless technologies mentioned above. As described in more detail herein, the remote computing system 808 may be configured to provide (e.g., visually display and/or aurally provide) the at least one of the patient biometrics and the associated information to a healthcare professional (e.g., a physician).

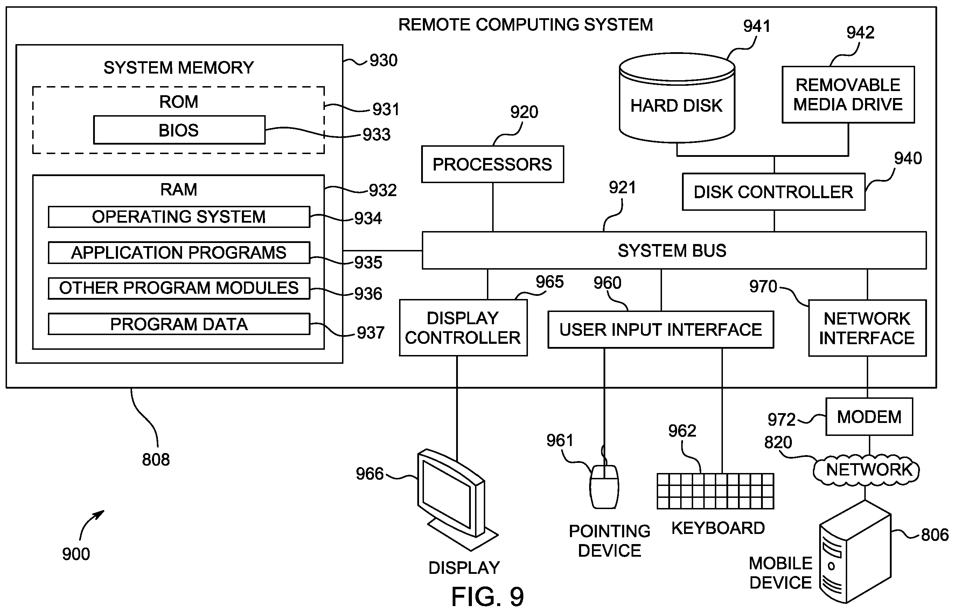

FIG. 9 is a system diagram of an example of a computing environment 900 in communication with network 820. In some instances, the computing environment 900 is incorporated in a public cloud computing platform (such as Amazon Web Services or Microsoft Azure), a hybrid cloud computing platform (such as HP Enterprise OneSphere) or a private cloud computing platform.

As shown in FIG. 9, computing environment 900 includes remote computing system 808 (hereinafter computer system), which is one example of a computing system upon which embodiments described herein may be implemented.

The remote computing system 808 may, via processors 920, which may include one or more processors, perform various functions. The functions may include analyzing monitored patient biometrics and the associated information and, according to physician-determined or algorithm driven thresholds and parameters, providing (e.g., via display 966) alerts, additional information or instructions. As described in more detail below, the remote computing system 808 may be used to provide (e.g., via display 966) healthcare personnel (e.g., a physician) with a dashboard of patient information, such that such information may enable healthcare personnel to identify and prioritize patients having more critical needs than others.

As shown in FIG. 9, the computer system 910 may include a communication mechanism such as a bus 921 or other communication mechanism for communicating information within the computer system 910. The computer system 910 further includes one or more processors 920 coupled with the bus 921 for processing the information. The processors 920 may include one or more CPUs, GPUs, or any other processor known in the art.

The computer system 910 also includes a system memory 930 coupled to the bus 921 for storing information and instructions to be executed by processors 920. The system memory 930 may include computer readable storage media in the form of volatile and/or nonvolatile memory, such as read only system memory (ROM) 931 and/or random access memory (RAM) 932. The system memory RAM 932 may include other dynamic storage device(s) (e.g., dynamic RAM, static RAM, and synchronous DRAM). The system memory ROM 931 may include other static storage device(s) (e.g., programmable ROM, erasable PROM, and electrically erasable PROM). In addition, the system memory 930 may be used for storing temporary variables or other intermediate information during the execution of instructions by the processors 920. A basic input/output system 933 (BIOS) may contain routines to transfer information between elements within computer system 910, such as during start-up, that may be stored in system memory ROM 931. RAM 932 may contain data and/or program modules that are immediately accessible to and/or presently being operated on by the processors 920. System memory 930 may additionally include, for example, operating system 934, application programs 935, other program modules 936 and program data 937.

The illustrated computer system 910 also includes a disk controller 940 coupled to the bus 921 to control one or more storage devices for storing information and instructions, such as a magnetic hard disk 941 and a removable media drive 942 (e.g., floppy disk drive, compact disc drive, tape drive, and/or solid state drive). The storage devices may be added to the computer system 910 using an appropriate device interface (e.g., a small computer system interface (SCSI), integrated device electronics (IDE), Universal Serial Bus (USB), or FireWire).

The computer system 910 may also include a display controller 965 coupled to the bus 921 to control a monitor or display 966, such as a cathode ray tube (CRT) or liquid crystal display (LCD), for displaying information to a computer user. The illustrated computer system 910 includes a user input interface 960 and one or more input devices, such as a keyboard 962 and a pointing device 961, for interacting with a computer user and providing information to the processor 920. The pointing device 961, for example, may be a mouse, a trackball, or a pointing stick for communicating direction information and command selections to the processor 920 and for controlling cursor movement on the display 966. The display 966 may provide a touch screen interface that may allow input to supplement or replace the communication of direction information and command selections by the pointing device 961 and/or keyboard 962.

The computer system 910 may perform a portion or each of the functions and methods described herein in response to the processors 920 executing one or more sequences of one or more instructions contained in a memory, such as the system memory 930. Such instructions may be read into the system memory 930 from another computer readable medium, such as a hard disk 941 or a removable media drive 942. The hard disk 941 may contain one or more data stores and data files used by embodiments described herein. Data store contents and data files may be encrypted to improve security. The processors 920 may also be employed in a multi-processing arrangement to execute the one or more sequences of instructions contained in system memory 930. In alternative embodiments, hard-wired circuitry may be used in place of or in combination with software instructions. Thus, embodiments are not limited to any specific combination of hardware circuitry and software.

As stated above, the computer system 910 may include at least one computer readable medium or memory for holding instructions programmed according to embodiments described herein and for containing data structures, tables, records, or other data described herein. The term computer readable medium as used herein refers to any non-transitory, tangible medium that participates in providing instructions to the processor 920 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 941 or removable media drive 942. Non-limiting examples of volatile media include dynamic memory, such as system memory 930. Non-limiting examples of transmission media include coaxial cables, copper wire, and fiber optics, including the wires that make up the bus 921. Transmission media may also take the form of acoustic or light waves, such as those generated during radio wave and infrared data communications.

The computing environment 900 may further include the computer system 910 operating in a networked environment using logical connections to local computing device 806 and one or more other devices, such as a personal computer (laptop or desktop), mobile devices (e.g., patient mobile devices), a server, a router, a network PC, a peer device or other common network node, and typically includes many or all of the elements described above relative to computer system 910. When used in a networking environment, computer system 910 may include modem 972 for establishing communications over a network 820, such as the Internet. Modem 972 may be connected to system bus 921 via network interface 970, or via another appropriate mechanism.

Network 820, as shown in FIGs. 8 and 9, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 910 and other computers (e.g., local computing device 806).

Any of the functions and methods described herein can be implemented in a general-purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer-readable media). The results of such processing can be maskworks that are then used in a semiconductor manufacturing process to manufacture a processor which implements features of the disclosure.

Any of the functions and methods described herein can be implemented in a computer program, software, or firmware incorporated in a non-transitory computer-readable storage medium for execution by a general-purpose computer or a processor. Examples of non-transitory computer-readable storage mediums include a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. Although features and elements are described above in particular combinations, each feature or element can be used alone without the other features and elements or in various combinations with or without other features and elements.

## Claims

1. A method for analyzing a procedure, the method comprising:
receiving an image of a portion of a body anatomy of a patient and registering the image to patient coordinates;
generating a procedure report based on a procedure comprising inserting a probe into the portion of the body anatomy, the probe comprising a tool tip configured to identify the position of the probe relative to the registered image and navigating the probe within the portion of the body anatomy, the procedure report comprising one or more components related to the procedure, the one or more components comprising a visual indication (320, 504, 507) of areas of the portion of the body anatomy navigated to by the probe;
and
providing the procedure report in a selected format; **characterised in that** the one or more components further comprises a three dimensional visual case overview (624), wherein the three dimensional visual case overview enables a user to rotate through different views of the body anatomy such that the probe and/or the body anatomy is visible from different viewpoints.

2. The method of claim 1, wherein the one or more components are further selected from at least one of the image, information related to registering the image to patient coordinates, a navigation procedure, a type of probe, and a procedure conclusion.

3. The method of claim 1, further comprising:
receiving a point of interest within the body anatomy; and
determining, from the procedure report, that the probe navigated to the point of interest during the procedure.

4. The method of claim 1, wherein the procedure report is provided in a timeline format segmented based on at least one of a user action and a system status.

5. The method of claim 4, wherein the timeline format segmented based on user action comprises providing a set of data corresponding to a point in time during the procedure, the point in time determined by a selected user action from a plurality of user actions during the procedure.

6. The method of claim 4, wherein the timeline format segmented based on system status comprises providing a set of data corresponding to a point in time during the procedure, the point in time determined by a selected system status from a plurality of system statuses during the procedure.

7. The method of claim 1, wherein the procedure is an Ear Nose and Throat procedure.

8. The method of claim 1, wherein the image is one of a computed tomography image and a magnetic resonance image.

9. The method of claim 1, wherein registering the image to patient coordinates comprises aligning the image to anatomical reference points at a current position of the patient.

10. The method of claim 1, wherein the tool tip comprises a magnetic sensor.

11. The method of claim 1, wherein the selected format is determined based on a predetermined preference.

12. The method of claim 1, wherein providing the procedure report comprises providing the report to an external system.

13. The method of claim 1, wherein the procedure report comprises a comparison to at least one of one or more previous procedures and a recognized standard.

14. A system comprising:
a probe, the probe configured to navigate at least a portion of a patient's body, the probe comprising a tool tip configured to identify the position of the probe relative to the registered image;
a processor, the processor configured to perform the steps of any one of claims 1 to 13:

15. The system of claim 14, further comprising a display, wherein the procedure report is provided, via the display, in a timeline format segmented based on at least one of a user action and a system status.

## Patentansprüche

1. Verfahren zum Analysieren eines Eingriffs, das Verfahren umfassend:
Empfangen eines Bilds eines Abschnitts einer Körperanatomie eines Patienten und Registrieren des Bilds mit Patientenkoordinaten;
Erzeugen eines Eingriffsberichts basierend auf einem Eingriff, umfassend ein Einführen einer Sonde in den Abschnitt der Körperanatomie, die Sonde umfassend eine Werkzeugspitze, die konfiguriert ist, um die Position der Sonde relativ zu dem registrierten Bild zu identifizieren und die Sonde innerhalb des Abschnitts der Körperanatomie zu navigieren, der Eingriffsbericht umfassend eine oder mehrere Komponenten, die sich auf den Eingriff beziehen, die eine oder die mehreren Komponenten umfassend eine visuelle Angabe (320, 504, 507) von Bereichen des Abschnitts der Körperanatomie, zu denen durch die Sonde navigiert wird; und
Bereitstellen des Eingriffsberichts in einem ausgewählten Format;
**dadurch gekennzeichnet, dass** die eine oder die mehreren Komponenten ferner eine dreidimensionale visuelle Fallübersicht (624) umfassen, wobei die dreidimensionale visuelle Fallübersicht es einem Benutzer ermöglicht, durch unterschiedliche Ansichten der Körperanatomie derart zu rotieren, dass die Sonde und/oder die Körperanatomie aus unterschiedlichen Blickwinkeln sichtbar ist.

2. Verfahren nach Anspruch 1, wobei die eine oder die mehreren Komponenten ferner aus mindestens einem von dem Bild, Informationen bezüglich einem Registrieren des Bilds mit Patientenkoordinaten, einem Navigationseingriff, einer Sondenart und einem Eingriffsabschluss ausgewählt werden.

3. Verfahren nach Anspruch 1, ferner umfassend:
Empfangen eines Punkts von Interesse innerhalb der Körperanatomie; und
Bestimmen, anhand des Eingriffsberichts, dass die Sonde während des Eingriffs zu dem Punkt von Interesse navigiert wird.

4. Verfahren nach Anspruch 1, wobei der Eingriffsbericht in einem Zeitleistenformat bereitgestellt wird, das basierend auf mindestens einem von einer Benutzeraktion und einem Systemstatus segmentiert wird.

5. Verfahren nach Anspruch 4, wobei das Zeitleistenformat, das basierend auf Benutzeraktionen segmentiert wird, das Bereitstellen eines Datensatzes umfasst, der einem Zeitpunkt während des Eingriffs entspricht, wobei der Zeitpunkt durch eine ausgewählte Benutzeraktion aus einer Vielzahl von Benutzeraktionen während des Eingriffs bestimmt wird.

6. Verfahren nach Anspruch 4, wobei das Zeitleistenformat, das basierend auf dem Systemstatus segmentiert wird, das Bereitstellen eines Datensatzes umfasst, der einem Zeitpunkt während des Eingriffs entspricht, wobei der Zeitpunkt durch einen ausgewählten Systemstatus aus einer Vielzahl von Systemstatus während des Eingriffs bestimmt wird.

7. Verfahren nach Anspruch 1, wobei der Eingriff ein Hals-Nasen-Ohren-Eingriff ist.

8. Verfahren nach Anspruch 1, wobei das Bild eines von einem Computertomographiebild oder einem Magnetresonanzbild ist.

9. Verfahren nach Anspruch 1, wobei das Registrieren des Bilds mit Patientenkoordinaten ein Ausrichten des Bilds mit anatomischen Referenzpunkten an einer aktuellen Position des Patienten umfasst.

10. Verfahren nach Anspruch 1, wobei die Werkzeugspitze einen Magnetsensor umfasst.

11. Verfahren nach Anspruch 1, wobei das ausgewählte Format basierend auf einer zuvor bestimmten Präferenz bestimmt wird.

12. Verfahren nach Anspruch 1, wobei das Bereitstellen des Eingriffsberichts das Bereitstellen des Berichts an ein externes System umfasst.

13. Verfahren nach Anspruch 1, wobei der Eingriffsbericht einen Vergleich mit mindestens einem oder mehreren vorherigen Eingriffen und einem anerkannten Standard umfasst.

14. System, umfassend:
eine Sonde, wobei die Sonde konfiguriert ist, um mindestens einen Abschnitt eines Körpers des Patienten zu navigieren, die Sonde umfassend eine Werkzeugspitze, die konfiguriert ist, um die Position der Sonde relativ zu dem registrierten Bild zu identifizieren;
einen Prozessor, wobei der Prozessor konfiguriert ist, um die Schritte nach einem der Ansprüche 1 bis 13 durchzuführen:

15. System nach Anspruch 14, ferner umfassend eine Anzeige, wobei der Eingriffsbericht über die Anzeige in einem Zeitleistenformat bereitgestellt wird, das basierend auf mindestens einem von einer Benutzeraktion und einem Systemstatus segmentiert wird.

## Revendications

1. Procédé permettant d'analyser une procédure, le procédé comprenant :
la réception d'une image d'une partie d'une anatomie corporelle d'un patient et la mise en correspondance de l'image avec des coordonnées de patient ;
la génération d'un rapport de procédure en fonction d'une procédure comprenant l'insertion d'une sonde dans la partie de l'anatomie corporelle, la sonde comprenant une pointe d'outil configurée pour identifier la position de la sonde par rapport à l'image mise en correspondance, et la navigation de la sonde au sein de la partie de l'anatomie corporelle, le rapport de procédure comprenant un ou plusieurs composants apparentés à la procédure, le ou les composants comprenant une indication visuelle (320, 504, 507) de zones de la partie de l'anatomie corporelle faisant l'objet d'une navigation par la sonde ; et
la fourniture du rapport de procédure sous un format sélectionné ;
**caractérisé en ce que** le ou les composants comprennent en outre un aperçu visuel de cas en trois dimensions (624), dans lequel l'aperçu visuel de cas en trois dimensions permet à un utilisateur de mettre en rotation des vues différentes de l'anatomie corporelle de telle sorte que la sonde et/ou l'anatomie corporelle sont visibles à partir de différents points de vue.

2. Procédé selon la revendication 1, dans lequel le ou les composants sont en outre sélectionnés parmi au moins l'un parmi l'image, des informations apparentées à la mise en correspondance de l'image avec des coordonnées de patient, une procédure de navigation, un type de sonde et une conclusion de procédure.

3. Procédé selon la revendication 1, comprenant en outre :
la réception d'un point d'intérêt au sein de l'anatomie corporelle ; et
la détermination, à partir du rapport de procédure, que la sonde a navigué vers le point d'intérêt pendant la procédure.

4. Procédé selon la revendication 1, dans lequel le rapport de procédure est fourni dans un format chronologique segmenté en fonction d'au moins l'un parmi une action d'utilisateur et un statut de système.

5. Procédé selon la revendication 4, dans lequel le format chronologique segmenté en fonction d'une action d'utilisateur comprend la fourniture d'un ensemble de données correspondant à un point dans le temps pendant la procédure, le point dans le temps étant déterminé par une action d'utilisateur sélectionnée parmi une pluralité d'actions d'utilisateur pendant la procédure.

6. Procédé selon la revendication 4, dans lequel le format chronologique segmenté en fonction d'un statut de système comprend la fourniture d'un ensemble de données correspondant à un point dans le temps pendant la procédure, le point dans le temps étant déterminé par un statut de système sélectionné parmi une pluralité de statuts de système pendant la procédure.

7. Procédé selon la revendication 1, dans lequel la procédure est une procédure d'oto-rhino-laryngologie.

8. Procédé selon la revendication 1, dans lequel l'image est l'une parmi une image de tomodensitométrie et une image de résonance magnétique.

9. Procédé selon la revendication 1, dans lequel la mise en correspondance de l'image avec des coordonnées de patient comprend l'alignement de l'image avec des points de référence anatomiques au niveau d'une position actuelle du patient.

10. Procédé selon la revendication 1, dans lequel la pointe d'outil comprend un capteur magnétique.

11. Procédé selon la revendication 1, dans lequel le format sélectionné est déterminé en fonction d'une préférence prédéterminée.

12. Procédé selon la revendication 1, dans lequel la fourniture du rapport de procédure comprend la fourniture du rapport à un système externe.

13. Procédé selon la revendication 1, dans lequel le rapport de procédure comprend une comparaison à au moins l'un parmi une ou plusieurs procédures précédentes et une norme reconnue.

14. Système comprenant :
une sonde, la sonde étant conçue pour naviguer dans au moins une partie du corps d'un patient, la sonde comprenant une pointe d'outil configurée pour identifier la position de la sonde par rapport à l'image mise en correspondance ;
un processeur, le processeur étant configuré pour mettre en œuvre les étapes de l'une quelconque des revendications 1 à 13 :

15. Système selon la revendication 14, comprenant en outre un affichage, dans lequel le rapport de procédure est fourni, par l'intermédiaire de l'affichage, sous un format chronologique segmenté en fonction d'au moins l'un parmi une action d'utilisateur et un statut de système.
